# EUROPEAN PATENT SPECIFICATION

(11) **EP 1 741 400 B1**
(45) Date of publication and mention of the grant of the patent: **10.09.2008**
(21) Application number: 06014135.5
(22) Date of filing: 07.07.2006
(51) Int. Cl.: A61B 17/86

(54) **Bone anchoring element**
Knochen- Verankerungselement
Elément d'ancrage dans l'os

(30) Priority: 08.07.2005 EP 05014839
(43) Date of publication of application: 10.01.2007
(73) Proprietor: BIEDERMANN MOTECH GmbH, 78054 VS-Schwenningen (DE)
(72) Inventor: Matthis, Wilfried, 79367 Weisweil (DE); Biedermann, Lutz, 78048 VS-Villingen (DE)
(74) Representative: Hofer, Dorothea

(56) References cited:
- US-A- 4 976 715
- US-A- 5 690 649
- US-A- 5 814 071
- US-A1- 2004 122 431
- US-B1- 6 187 009
- US-B1- 6 375 458

## Description

The present invention relates to a bone anchoring element.

A known form of a bone anchoring element is a bone screw comprising a shaft with a thread for screwing the screw into a bone. The bone screw is manually inserted into the bone by means of a screw driver, which is a time-consuming and force-requiring process. Moreover, during the process in which the screw is inserted into the bone, high pressure forces may be acting on the bone itself, which is undesirable in certain clinical applications such as e.g., in neuro surgery, spinal surgery, pediatric surgery or trauma surgery.

EP 0 714 643 A1 discloses a bone fixation device such as a screw or a pin which has a micro-textured contact surface to enhance the installation or gripping characteristics of the device. The micro-textured contact surface includes e.g. angled rasp teeth, which bite or flex to resist movement in one direction and yet flex or slide to allow a relatively easy contact movement in the other direction.

DE 198 01 219 A1 discloses a bone nail having rigid barb-like projections being arranged in circumferential rows around the nail. The barb-like projection has a saw-tooth shape which facilitates insertion of the nail as well as prevents loosening of the nail. However, a removal of the nail without destroying the bone is not possible.

CH 682450 A5 discloses an anchoring nail for the fixation of orthopedic bone implants. The nail consists of a head part and a shaft part, the shaft having retention elements provided on its outer wall which are arranged along a helical line. The retention elements are wedge-shaped and are provided with cutting edges which allow to screw out and remove the nail from the bone material. However, the core hole which has to be drilled in advance to allow an easy insertion of the nail into the bone and a removal of the nail has to have a precise diameter. In addition, the wedge-shape of the retention elements per se does not allow an easy insertion.

US 5,814,071 discloses a suture anchor assembly which includes an elongated insertion stem and an approximately cylindrical anchoring element having an axial channel for receiving the insertion stem. The proximal end of the anchoring element is capable of telescoping or camming movement over the distal end of an expander element so that resilient proximal portions of the anchoring element are forced outward against the expander element, causing the resilient proximal portions of the anchoring element to expand into the bone hole, fixing the anchoring element in a pressure fit firmly within the opening.

US 6,375,458 B1 describes the fabrication of orthodontic and medical instruments and devices and components thereof from a specially processed nickel-titanium shape memory alloy. The process results in a ductile alloy for ease of forming and a heat treatment which renders the fabricated orthodontic and medical component highly elastic, with a high resistance to kinking and with good corrosion resistance.

It is therefore an object of the invention to provide a bone anchoring element, which can be inserted into the bone more rapidly, more easily and with less force than conventional bone screws and nails. It also desirable to provide a bone anchoring element that is versatile and useful in many clinical requirements and which is easy to manufacture. Preferably, the bone anchoring element does not exert damaging forces on the bone during insertion, provides for secure attachment, and then can be further inserted or can be removed in a screw-like fashion. It is a further object of the invention to provide a method for manufacturing such a bone anchoring element.

The above mentioned object of the invention is solved by a bone anchoring element according to claim 1 or by a method according to claim 12. Further developments are given in the dependent claims.

The bone anchoring element according to the invention facilitates rapid and secure anchoring to the bone by pressing the bone anchoring element into a prepared core hole in the bone. The barb elements are arranged on at least on one helical line around the shaft axis of a tubular body part of the shaft of the bone anchoring element. The barb elements provide for a thread-like function, which allows to correct the position of the bone anchoring element in the core hole after inserting it into the core hole, by either positioning it deeper into the bone by means of a screwing-inwards motion or by screwing it backward. The barb elements prevent the bone anchoring element from being pulled out or coming loose. The bone anchoring element of the present invention can be removed, if required, like a screw by turning it in the opposite or counterclockwise direction from which it was inserted.

The bone anchoring element according to the invention is easy to manufacture.

If the tubular body of the shaft is made of a shape memory alloy, the shape memory effect can be used in such a way that the barb elements do not project during insertion of the bone anchoring element into the bone and rise up when the bone anchoring element is inserted due to the action of the body heat. The tubular body can also be made of a material having superelastic properties or a spring like behaviour. For example a titanium alloy having superelasticity or stainless steel can be used.

Further features and advantages of the invention will become apparent and will be best understood by reference to the following detailed description taken in conjunction with the accompanying drawings.
- Fig. 1: shows a perspective exploded view of a bone anchoring element according to a first embodiment.
- Fig. 2: shows an enlarged part of a cross-sectional view through the wall of the tubular body of the bone anchoring element of Fig. 1 in a longitudinal direction.
- Fig. 3: shows a side view of the bone anchoring element according to Fig. 1 in an assembled state.
- Fig. 4: shows an enlarged cross-sectional view of the bone anchoring element according to Fig. 3 along the line A-A.
- Fig. 5: shows a schematic view of a first step of the insertion of the bone anchoring element according to Fig. 1 to 4.
- Fig. 6: shows a schematic view of the inserted state of the bone anchoring element according to Fig. 5.
- Fig. 7: shows a perspective view of a second embodiment of the bone anchoring element.
- Fig. 8: shows a third embodiment of the bone anchoring element.
- Fig. 9: shows an exploded view of a fourth embodiment of the bone anchoring element.

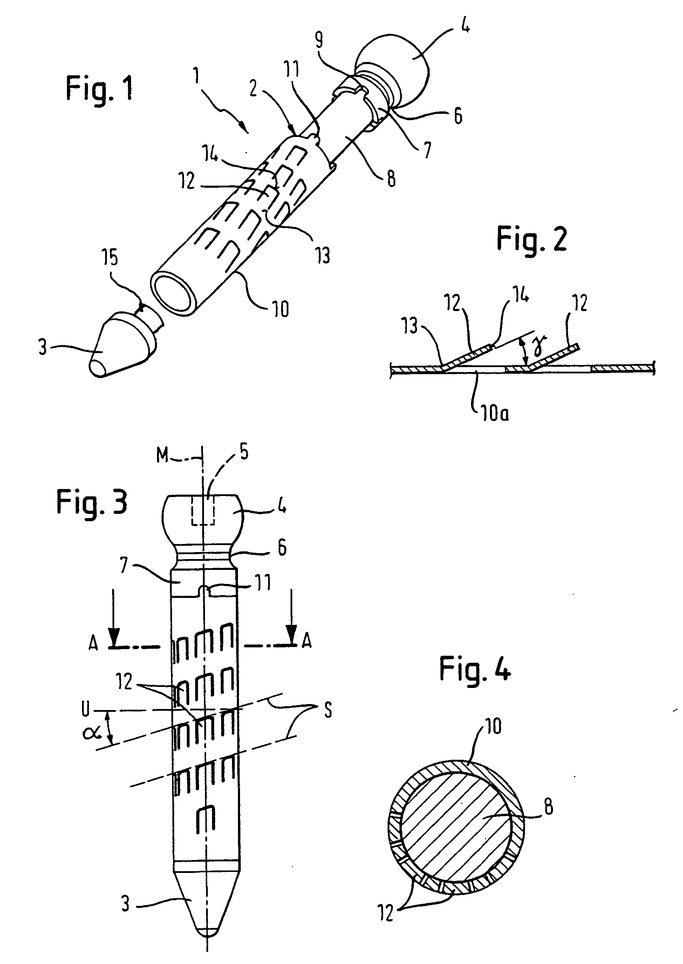

A bone anchoring element according to a first embodiment of the invention is described with reference to Fig. 1 to 4. The bone anchoring element 1 comprises a shaft 2 with a tip 3 at one end and a head 4 at the other end. The head 4 is spherical segment-shaped and has on its free end a recess 5 for engagement with a screwing-in tool. Between the head 4 and the shaft 2 is a neck portion 6 with a circumferentially projecting shoulder 7 at the side opposite to the spherical head 4. The shoulder 7 has an outer diameter which is larger than the neck portion 6 and slightly smaller than the diameter of the head 4. Adjacent to the shoulder 7 a cylindrical first shaft part 8 is provided. The diameter of the cylindrical first shaft part 8 is smaller than the diameter of the shoulder. At the edge of the shoulder 7, facing the shaft part 8, a plurality of U-shaped recesses are provided equidistantly in a circumferential direction which are open towards the side of the cylindrical shaft part 8.

The shaft 2 further consists of a tubular body 10 which has an inner diameter slightly larger than the outer diameter of the cylindrical shaft part 8 so that the tubular body 10 can be placed in a sliding motion onto the cylindrical shaft part 8. The outer diameter of the tubular body 10 corresponds to the outer diameter of the shoulder 7 so that when the tubular body 10 is placed onto the cylindrical shaft part 8 the outer surface of the tubular body 10 is flush with the outer surface of the shoulder 7. On its end facing the head 4 the tubular body 10 has projections 11 which correspond in their shape and their arrangement to the U-shaped recesses 9 provided at the shoulder 7 for engagement when the tubular body 10 is fully placed onto the cylindrical shaft part 8. In this assembled state the end of the tubular body abuts the free end of the shoulder 7.

The tubular body 10 comprises a plurality of barb elements 12. The barb elements 12 are formed by substantially parallelogram-shaped cuts 10a being made in the wall of the tubular body 10. The end base 13 of the parallelogram-shaped cuts 10a is not cut-out from the tubular body and acts as the attachment and the bending side for the barb elements 12 in the wall of the tubular body. The barb elements 12 are preferably arranged such that when the tubular body is slid onto the cylindrical shaft part 8, the end base 13 of the barb elements 12 face the tip 3 while the free end face the head 4. As can be seen particularly in Fig. 3, the barb elements 12 are arranged on a helical line S around the shaft axis M. The free ends 14 of the barb elements are inclined by an angle α with regard to the circular circumference line U, the angle α corresponding to the helical angle of the helical line S. Thus, the free ends 14 of the barb element form cutting edges similar to the crest of a screw thread.

As can be seen in particular from Fig. 2, the barb elements 12 project from the surface of the tubular body 10 by an angle γ which is selected during the manufacturing process based upon the material used and the actual dimensions of the barb elements 12 so that a desired stiffness of the barb element is obtained. Due to their configuration and attachment onto the wall of the tubular body 10, the barb elements 12 are elastically deformable relative to the tubular body 10. When the barb elements 12 are collapsed or pressed into the cuts, they are pre-tensioned.

The axial length of the tubular body 10 corresponds to the length of the cylindrical shaft part 8 so that in an assembled state a free end of the cylindrical shaft part is flush with the free end of the tubular body 10. The cylindrical shaft part 8 has a cylindrical recess (not shown) at its free end to receive a correspondingly shaped cylindrical projection 15 provided at the tip 3 in a press-fit manner. The outer diameter of the base of the tip 3 corresponds to the outer diameter of the tubular body 10 so that in an assembled state as shown in Fig. 3 the base of the tip 3 is flush with the outer wall of the tubular body 10.

The bone anchoring element can be made of any body-compatible material. Preferably, a body-compatible metal, such as titanium, stainless steel and their alloys, or a body-compatible plastic material can be used. The tubular body 10 having the barb elements 12 is made of a shape memory alloy having shape memory characteristics. For example, nickel titanium alloys such as nitinol are suitable for use for the tubular body 10.

In operation, the bone anchoring element 1 is initially preassembled by sliding the tubular body 10 onto the cylindrical shaft part 8 such that the projections 11 come into engagement with the recesses 9. Thus, a rotation of the tubular body 10 on the cylindrical shaft part 8 is prevented. Thereafter, the tip 3 is firmly connected with the cylindrical shaft part 8.

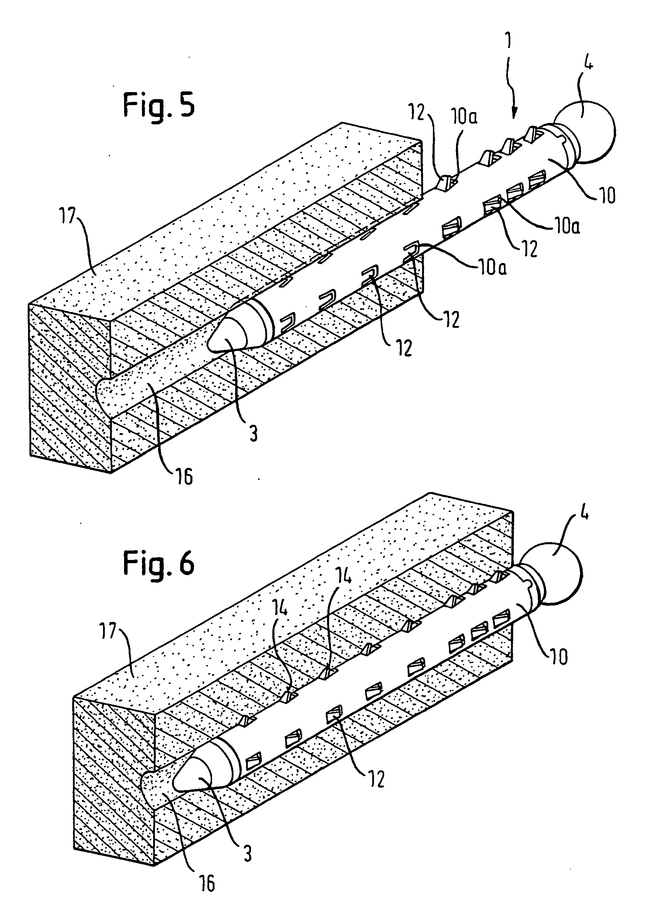

In use, as is shown schematically in Figs. 5 and 6, first a core hole 16 is prepared in the bone 17. The diameter of the core hole corresponds essentially to the outer diameter of the tubular body 10 or it can be slightly larger or smaller, depending upon the desired result or circumstances. The diameter of the core hole 16 is selected depending on the diameter of the tubular body and the flexibility of the barb elements 12 so that the desired resistance is provided by the barb elements 12. The selected diameter depends also upon the bone quality, for example, it can be selected larger for healthy hard bone and smaller for osteoporotic weak bone. Subsequently, as shown in Fig. 5, the bone anchoring element 1 is inserted into the core hole 16. As the bone anchoring element is being inserted into the core hole 16 of the bone 17, the barb elements 12 are in a collapsed state and are pressed against or into the cuts due to their elasticity. The sliding motion enables the bone anchoring element to be inserted rapidly and in a smooth way, in contrast to the conventional bone screws using the screwing-in-process. When inserted, the pre-tensioned barb elements 12 expand and rise up and press with their cutting edge 14 outwardly against the wall of the core hole 16, as is evident from Fig. 6. The barb elements 12 prevent the bone anchoring element from being pulled out or from falling out of the core hole 16.

For further and/or final positioning of the bone anchoring element in the core hole 16 or for positioning of the head 4, the bone anchoring element is screwed further into the core hole 16 or screwed out therefrom, like a screw, by means of a turning motion with the screwing-in tool engaging the recess 5 in the head 4. During the process, the cutting edges 14 of the barb elements, being positioned on the helical line S, act like the crest of a thread. The bone anchoring element can be removed just like a bone screw by turning it in a counterclockwise direction.

In most cases, a conventional bone screw requires not only that the core hole is drilled into the bone, but also that the bone threads are pre-cut into the bone. In all cases with the insertion of a conventional bone screw, a repeated turning motion is required. Compared to the time required to anchor a conventional bone screw, the time required to insert the bone anchoring element according to the instant invention is substantially shorter due to the fact that the bone anchoring element slides or glides into the core hole 16 without having to encounter the forces of the screw thread on the core hole 16 and the bone 17. Nonetheless, because of its design and configuration, the bone anchoring element does not fall out of the core hole 16. Also, when a conventional bone screw is inserted it cuts with the thread crest in the bone material.

A process for manufacturing the bone anchoring element comprises providing a cylinder having a diameter corresponding to that of the tubular body 10, the cylinder being made of a shape memory alloy. In a next step, a coaxial bore is provided in the cylinder such that a tubular body is prepared.

Thereafter, the barb elements 12 are generated by means of cutting, for example laser cutting, parallelogram-shaped cuts in the wall of the tubular body in which one side which shall be the base of the barb elements is not cut out. Thereafter the barb elements 12 are bent such that they project to the outside.

Modifications of the first embodiment are possible. The head 4 needs not to be spherical segment-shaped but can have another shape, in particular it can have any shape of known screw heads. The recesses 9 and the corresponding projections 11 need not to be U-shaped but can have a different shape. At least one recess and a corresponding projection is necessary to provide securing against rotation. However, it is also possible to prevent rotation by different means. For example, a pin which can be inserted into a transverse bore provided in the tubular body 10 and the cylindrical shaft part 8 can be used.

The barb elements 12 need not to have a parallelogram-shape, but can have another shape, as long as the cutting edges lie on a helical line. For example, the barb elements can have a trapezoidal shape. The pitch of the helical line may vary along the length of the tubular body 10. The barb elements 12 need not to be provided over the whole length of the tubular body 10 but can be provided also only in a section of the tubular body 10. The distance between the barb elements may also vary.

In yet another modification of the invention, the tubular body 10 is treated before assembly of the bone anchoring element in such a way that the barb elements project at a body temperature or at an elevated temperature and are in a collapsed position at a lower temperature, e.g. at room temperature. In operation the bone anchoring element with collapsed barb elements is pressed into the core hole. After the bone anchoring element warms up and equilibrates to the body temperature or is heated through an external device, the barb elements expand to their final position. This provides the advantage that it reduces the amount of force that is required to press the bone anchoring element into the core hole 16 and enables the adjustment of the bone anchoring element to a desired depth by the sliding motion during its insertion as long as the barb elements are in a collapsed state and do not press against the wall of the core hole 16. If the tubular body 10 has super elasticity in addition to shape memory characteristics, the higher elasticity of the barb elements simplifies the handling and provides additional security in anchoring the bone anchoring element in the bone.

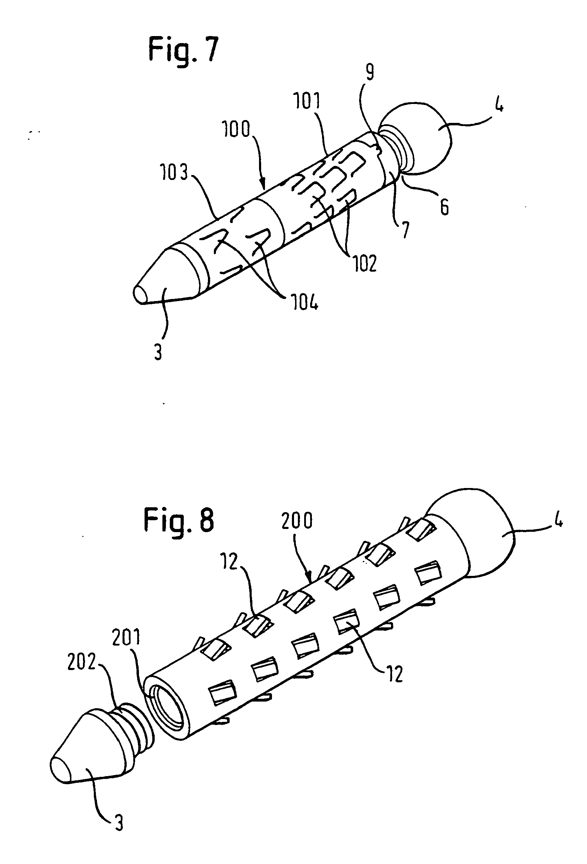

In the second embodiment shown in Fig 7, those parts which correspond to the parts of the first embodiment are characterized with the same reference numerals. The bone anchoring element of the second embodiment differs from the bone anchoring element according to the first embodiment in that the tubular body 100 comprises two sections. A first section 101 with barb elements 102 having a first distance from each other in a circumferential direction and having substantially parallelogram-shape and a second section 103 having barb elements 104 which have a second distance from each other in the circumferential direction which is larger than the first distance of the barb elements 102 in the first section 101. Hence, the barb elements 104 in section 103 are less densely arranged than the barb elements 102 in section 101. The barb elements 104 are substantially V-shaped. The bone anchoring element is particularly suitable for application in the spinal column, in particular for anchoring in the vertebra. The second section 103 with the larger distance between the barb elements is provided adjacent to the tip 3 and comes into engagement with the vertebral body. The first section 101 is adjacent to the head and comes into engagement with the pedicle. Thus, due to the larger distance between the barb elements in the vertebral body, any potential damage of the vertebral body can be minimized while providing higher locking forces of the barbs in the pedicle area.

In the embodiment shown in Fig. 7, the tubular body is made of one single tube. However, the tubular body with sections having different characteristics of the barb elements can be made of two separate tubular bodies. In such a case, it is necessary to prevent rotation between the two tubular bodies. This can be realized, for example, by providing recesses at the end of the first tubular body in which projections of the second tubular body engage.

Modifications are possible, for example, more than two sections with different characteristics of the barb element can be provided.

Fig. 8 shows a third embodiment of the invention. The shaft of the bone anchoring element is not composed of the cylindrical shaft part 8 and the tubular body 10 but consists of a tubular body 200 only. Thus, the shaft is hollow. At its end facing the tip 3 the tubular body 200 comprises a section with an inner thread 201 which cooperates with an outer thread 202 on the cylindrical projection of the tip 3. Alternatively, the tip 3 can also be connected in a press-fit manner to the tubular body 200. The tubular body 200 comprises the barb elements 12 as in the previous embodiments. Different sections with different shapes and/or distances of barb elements can be provided. Also, the pitch of the helical line S can vary along the length of the tubular body 200. The head 4 is either formed integrally with the tubular body 200 or comprises a projection with an outer thread which can be screwed into the other end of the tubular body 200 which has a corresponding inner thread. Alternatively, the head 4 can be connected in a press-fit manner to the tubular body 200.

The bone anchoring element according to the third embodiment is particularly simple to manufacture.

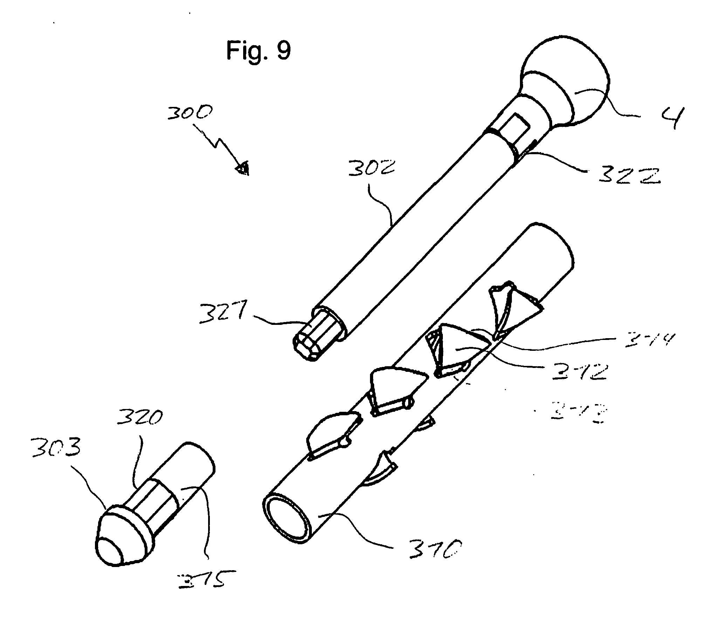

Fig. 9 shows an exploded view of a fourth embodiment of the disclosure. The bone anchoring element 300 according to Fig. 9 comprises a tip 303, a tubular body 310 and a shaft 302 provided with a head 4 at one end. The fourth embodiment differs from the above described first embodiment in that barb elements 312 are provided instead of the barb elements 12 of the first embodiment. Further, the fourth embodiment differs from the first embodiment in the structures for connecting the tip 303, the shaft 302 and the tubular body 310.

First, the barb elements 312 will be described with reference to Fig. 9. As can be seen in Fig. 9, in top view the barb elements 312 have the contour of an irregular quadrangle which is twisted so that it has a curvature. The free ends 314 of the barb elements 312 lie substantially on a helical line. The width of the free ends 314 of the barb elements 312 is larger than the width of the end base 313 of the barb elements 312. As a result, the width of the free ends 314 is enlarged as compared to first embodiment which provides a stable construction and an enlarged contact surface of the free ends 314 with the bone into which the bone anchoring element is to be inserted. The free ends 314 of the barb elements form a helical line around the axis of the shaft 302 with the pitch of the helical line enlarged as compared to the first embodiment. The large pitch of the helical line allows fast adjustment of the position of the bone anchoring element in the bone by further screwing-in into the bone or unscrewing from the bone, respectively.

As can be seen in Fig. 9, a first connecting structure 320 is provided on the projection 315 of tip 303. This first connecting structure 320 serves to cooperate with the inner bore of the tubular body 310 for establishing a fixation between the tip 303 and the tubular body 310. Further, a second connecting structure 321 is provided on the shaft 302 on the side directed towards the tip 303. This second connecting structure 321 cooperates with a corresponding connecting structure (not shown) provided in a concentric inner bore which is provided in the projection 315 of the tip 303 on the side directed towards the shaft 302 and serves to establish a fixation between the tip 303 and the shaft 302. Furthermore, a third connecting structure 322 is provided on the outer circumference of the shaft 302 on the side of the head 4. This third connecting structure 322 cooperates with the inner bore of the tubular body 310 on the side directed towards the head 4 in order to establish a fixation between the shaft 302 and the tubular body 310 on the side of the head 4.

In the embodiment shown, the first connecting structure 320 is provided by forming a part of the projection 315 with a substantially square cross-section with rounded edges. When fitted into the inner bore of the tubular body 320, this shape leads to a distortion-fit connection. The second connecting structure 321 is provided by forming an end of the shaft 302 with a substantially square cross-section with rounded edges. The corresponding connecting structure in the inner bore of the projection 315 is formed by a recess with a substantially square cross-section with rounded edges into which the second connecting structure 321 can be fitted such that positive locking as well as press-fit is achieved. Similar to the first connecting structure 320, the third connecting structure 322 is provided by forming a part of the shaft 302 on the side of the head 4 with a substantially square outer cross-section with rounded edges so that a distortion-fit connection to the tubular body can be achieved.

In the embodiment, the fixation of the tip 303 and the tubular body 310 is achieved by distortion locking, the fixation of the tubular body 310 and the shaft 302 on the side of the head 4 is achieved by distortion locking, and the fixation of the tip 303 and the shaft 302 is achieved by positive locking and frictional locking.

By providing the first connecting structure 320, the second connecting structure 321 and the third connecting structure 322 three sections for transmitting torsional forces are provided in the fourth embodiment. This results in improved properties during insertion into the bone and removal from the bone.

The shape of the first, the second and the third connecting structures is not limited to a square shape with rounded edges. For example, hexagonal shapes, octagonal shapes etc. with or without rounded edges are possible as well.

In all embodiments described above the diameter of the tubular body can vary over the axial length of the tubular body, for example it can decrease towards the tip so that the tubular body has a conical outer surface.

The bone anchoring element according to the invention can be used together with a plate to establish a bone fixing device or with a receiver part to be connected with a rod to establish a spinal fixation system. Also, all further applications are conceivable in which the bone anchoring element can be used instead of conventional bone screws or instead of conventional pins used in a bone anchoring manner.

## Claims

1. A bone anchoring element having a shaft for anchoring in a bone, wherein said shaft (2; 200) comprises a plurality of barb elements (12),
wherein said barb elements are integrally formed in a tubular body (10; 100; 200) which is part of said shaft, **characterised in that** said tubular body (10 ; 100; 200) is formed of a shape memory alloy.

2. The bone anchoring element of claim 1, wherein said barb elements (12) are cut out of the wall of said tubular body (10; 100; 200).

3. The bone anchoring element of claim 1 or 2, wherein the barb elements (12) have a free cutting edge (14), respectively, which includes an angle (α) with a circular circumference line.

4. The bone anchoring element of one of claims 1 to 3, wherein the barb elements (12) are elastically deformable relative to the wall of the tubular body (10; 100; 200).

5. The bone anchoring element of one of claims 1 to 4, wherein said tubular body (200) forms a hollow part of said shaft.

6. The bone anchoring element of one of claims 1 to 4, wherein said tubular body (10; 100) is placed onto an essentially cylindrical shaft basis (8).

7. The bone anchoring element of claim 6, wherein means (9, 11) for preventing said tubular body (10; 100; 201; 202) from rotating are provided.

8. The bone anchoring element of one of claims 1 to 7, wherein a tip (3) is provided which can be fixedly connected to said tubular body (10; 100; 200), preferably by a press-fit or a threaded connection.

9. The bone anchoring element of one of claims 1 to 8, wherein a head (4) is provided, which can be connected to said tubular body.

10. The bone anchoring element of one of claims 1 to 9, wherein said tubular body is formed from nitinol.

11. The bone anchoring element of one of claims 1 to 10, wherein the barb elements (12) are arranged along at least one helical line (S) around the shaft axis.

12. A method of manufacturing the bone anchoring element of any of claims 1 to 11,
comprising a step of providing a tubular body (10; 100; 200) formed of a shape memory alloy and
generating a plurality of barb elements (12) by cuts forming the shape of the barb elements in the wall of said tubular body.

13. The method of claim 12, wherein the barb elements are generated by laser cutting.

## Patentansprüche

1. Knochenverankerungselement mit einem Schaft zum Verankern in einem Knochen, wobei der Schaft (2; 200) eine Mehrzahl von Widerhakenelementen (12) aufweist,
wobei die Widerhakenelemente einstückig in einem rohrförmigen Körper (10; 100; 200) gebildet sind, der Teil des Schafts ist,
**dadurch gekennzeichnet, daß** der rohrförmige Körper (10; 100; 200) aus einer Formgedächtnislegierung gebildet ist.

2. Knochenverankerungselement nach Anspruch 1, wobei die Widerhakenelemente (12) aus der Wand des rohrförmigen Körpers (10; 100; 200) herausgeschnitten sind.

3. Knochenverankerungselement nach Anspruch 1 oder 2, wobei die Widerhakenelemente (12) jeweils eine freie Schneidkante (14) haben, die einen Winkel (α) mit einer kreisförmigen Umfangslinie einschließt.

4. Knochenverankerungselement nach einem der Ansprüche 1 bis 3, wobei die Widerhakenelemente (12) relativ zur Wand des rohrförmigen Körpers (10; 100; 200) elastisch deformierbar sind.

5. Knochenverankerungselement nach einem der Ansprüche 1 bis 4, wobei der-rohrförmige Körper (200) einen hohlen Teil des Schafts bildet.

6. Knochenverankerungselement nach einem der Ansprüche 1 bis 4, wobei der rohrförmige Körper (10; 100) auf einer im wesentlichen zylindrische Schaftbasis (8) plaziert ist.

7. Knochenverankerungselement nach Anspruch 6, wobei Mittel (9, 11) zum Verhindern des Rotierens des rohrförmigen Körpers (10; 100; 201; 202) vorgesehen sind.

8. Knochenverankerungselement nach einem der Ansprüche 1 bis 7, wobei eine Spitze (3) vorgesehen ist, die mit dem rohrförmigen Körper (10; 100; 200) fest verbunden werden kann, vorzugsweise durch eine Paßsitz- oder eine Gewindeverbindung.

9. Knochenverankerungselement nach einem der Ansprüche 1 bis 8, wobei ein Kopf (4) vorgesehen ist, der mit dem rohrförmigen Körper verbunden werden kann.

10. Knochenverankerungselement nach einem der Ansprüche 1 bis 9, wobei der rohrförmige Körper aus Nitinol gebildet ist.

11. Knochenverankerungselement nach einem der Ansprüche 1 bis 10, wobei die Widerhakenelemente (12) entlang wenigstens einer Schraubenlinie (S) um die Schaftachse angeordnet sind.

12. Verfahren zum Herstellen des Knochenverankerungselements nach einem der Ansprüche 1 bis 11, das den Schritt des Bereitstellens eines rohrförmigen Körpers (10; 100; 200) der aus einer Formgedächtnislegierung gebildet ist, und
das Erzeugen einer Mehrzahl von Widerhakenelementen (12) durch Einschnitte, die die Form der Widerhakenelemente bilden, in der Wand des rohrförmigen Körpers, beinhaltet.

13. Verfahren nach Anspruch 12, wobei die Widerhakenelemente durch Laserschneiden erzeugt werden.

## Revendications

1. Élément d'ancrage dans l'os ayant un arbre d'ancrage dans un os, dans lequel ledit arbre (2; 200) comprend une pluralité d'éléments à crans (12),
dans lequel lesdits éléments à crans sont intégralement formés dans un corps tubulaire (10; 100; 200) qui fait partie dudit arbre, **caractérisé en ce que** ledit corps tubulaire (10; 100; 200) est formé d'un alliage à mémoire de forme.

2. Élément d'ancrage dans l'os de la revendication 1, dans lequel lesdits éléments à crans (12) sont découpés de la paroi dudit corps tubulaire (10; 100; 200).

3. Élément d'ancrage dans l'os de la revendication 1 ou 2, dans lequel les éléments à crans (12) ont une arête de coupe libre (14), respectivement, qui inclut un angle (α) avec une ligne circonférentielle circulaire.

4. Élément d'ancrage dans l'os de l'une des revendications 1 à 3, dans lequel les éléments à crans (12) sont élastiquement déformables par rapport à la paroi du corps tubulaire (10; 100; 200).

5. Élément d'ancrage dans l'os de l'une des revendications 1 à 4, dans lequel ledit corps tubulaire (200) forme une partie creuse dudit arbre.

6. Élément d'ancrage dans l'os de l'une des revendications 1 à 4, dans lequel ledit corps tubulaire (10; 100) est placé sur une base d'arbre essentiellement cylindrique (8).

7. Élément d'ancrage dans l'os de la revendication 6, dans lequel des moyens (9, 11) pour empêcher la rotation dudit corps tubulaire (10; 100; 201; 202) sont prévus.

8. Élément d'ancrage dans l'os de l'une des revendications 1 à 7, dans lequel une pointe (3) est fournie qui peut être reliée de manière fixe audit corps tubulaire (10; 100; 200), de préférence par le biais d'une liaison par filetage ou un ajustement avec serrage.

9. Élément d'ancrage dans l'os de l'une des revendications 1 à 8, dans lequel une tête (4) est fournie, qui peut être raccordée audit corps tubulaire.

10. Élément d'ancrage dans l'os de l'une des revendications 1 à 9, dans lequel ledit corps tubulaire est formé de nitinol.

11. Élément d'ancrage dans l'os de l'une des revendications 1 à 10, dans lequel les éléments à crans (12) sont agencés le long d'au moins une ligne hélicoïdale (S) autour de l'axe de l'arbre.

12. Procédé de fabrication de l'élément d'ancrage dans l'os de l'une quelconque des revendications 1 à 11,
comprenant une étape consistant à prévoir un corps tubulaire (10; 100; 200) formé d'un alliage à mémoire de forme et
générer une pluralité d'éléments à crans (12) par des découpages formant la forme des éléments à crans dans la paroi dudit corps tubulaire.

13. Procédé de la revendication 12, dans lequel les éléments à crans sont générés par un découpage au laser.
